# EUROPEAN PATENT SPECIFICATION

(11) **EP 4 276 448 B1**
(45) Date of publication and mention of the grant of the patent: **02.12.2026**
(21) Application number: 23169806.9
(22) Date of filing: 25.04.2023
(51) Int. Cl.: G01N 21/65, G01N 21/85, B07C 5/342, B07C 5/36, G01J 3/44

(54) **MEASUREMENT APPARATUS AND SORTING APPARATUS FOR SORTING OBJECTS INCLUDING THE MEASUREMENT APPARATUS**
MESSVORRICHTUNG UND SORTIERVORRICHTUNG ZUM SORTIEREN VON OBJEKTEN MIT DER MESSVORRICHTUNG
APPAREIL DE MESURE ET APPAREIL DE TRIAGE POUR TRIER DES OBJETS COMPRENANT L'APPAREIL DE MESURE

(30) Priority: 12.05.2022 JP 2022078799
(43) Date of publication of application: 15.11.2023
(60) Divisional application: 26213590.8
(73) Proprietor: CANON KABUSHIKI KAISHA, Tokyo 146-8501 (JP)
(72) Inventor: KATO, Shigeki, Tokyo, 146-8501 (JP); SHIMADA, Takuya, Tokyo, 146-8501 (JP); UCHIYAMA, Shinzo, Tokyo, 146-8501 (JP)
(74) Representative: Canon Europe Limited

(56) References cited:
- DE-A1- 19 816 881
- JP-A- 2013 036 971
- US-A- 5 471 311
- US-A- 5 936 229
- DUPONT AURÉLIE ET AL: "Nanoscale three-dimensional single particle tracking", NANOSCALE, vol. 3, no. 11, 1 January 2011 (2011-01-01), United Kingdom, pages 4532, XP093085472, ISSN: 2040-3364, DOI: 10.1039/c1nr10989h

## Description

### BACKGROUND OF THE INVENTION

### Field of the Invention

The present invention relates to a measurement apparatus and a sorting apparatus for sorting objects using the measurement apparatus.

### Description of the Related Art

Conventionally, from the perspective of mass-productivity and component cost, molded resinous components have been widely used for structural components and exterior components of automobiles and electric appliances. The recent industrial world is facing an urgent need to give consideration to the environment, so that these molded resinous components are recycled and reused. In the conventional recycling, waste automobiles and waste home electronics are crushed to a size of 10 to 100 mm and collected as residual substances containing various types of plastics after the substances such as iron and aluminum are collected in the course of various types of recycling processing.

In many cases, these residual substances are eventually reused as the fuel burned through thermal recycling processing.

An attempt to conduct horizontal recycling where these residual substances such as plastic are reused as the materials of molded resinous components has been made, and a resin identification apparatus for identifying and sorting a particular type of plastic from various residual substances containing plastic has been developed.

As a measurement method for specifying a resin type in a non-contact state by emitting light, the following technique which specifies a resin type by using the Raman scattering has been discussed.

A resin identification apparatus discussed in Japanese Patent Application Laid-Open No. 2013-36971 moves illumination light parallel to a conveyer-belt when resin conveyed by the conveyer-belt is measured by the Raman spectroscopy, so that a strong Raman scattering signal can be acquired without damaging or denaturalizing the resin. While emission of the excitation laser light is being performed, the resin identification apparatus moves a lighting optical system parallel to an upper face of a placement table in a range greater than or equal to a spot diameter of the excitation laser light on the target object, or preferably, in a range greater than or equal to two to three times the spot diameter. In this way, the excitation laser light is not intensively emitted to one point of the target object, so that the resin identification apparatus can prevent the target object from being damaged or denaturalized.

According to the technique discussed in Japanese Patent Application Laid-Open No. 2013-36971, even when high-power laser is emitted to the resin, a resin type can be identified through the Raman measurement without denaturalizing the resin. Generally, it is necessary to measure an extremely faint signal through the Raman measurement. Therefore, the identification apparatus has to include a laser light source having a high-output power. However, when use of a low-cost mountable laser diode (LD) as a light source is considered, it is necessary to improve a signal-noise (S/N) ratio because there is a limit to the output power of the LD. In order to acquire a Raman signal of high S/N ratio, for example, when measurement is performed, signal intensity is increased by extending the exposure time spent at the sensor by reducing the belt speed, and noise is reduced through repetitive averaging.

On the other hand, throughput of the identification processing has to be enhanced because a huge amount of residual resinous substances of 1 t/h to 2 t/h are generated in the course of the recycling processing. Therefore, it is desirable to increase the speed of the conveyer-belt. If a speed of the conveyer-belt is increased in order to enhance the throughput, a period of time the sample exists just beneath the illumination light is shortened, so that the exposure time necessary to perform Raman measurement through the spectroscope cannot be ensured, and the number of times of repetitive averaging cannot be ensured. Therefore, it is difficult to ensure the S/N ratio.

As described above, there is an issue that sorting of resin cannot easily be executed while achieving both of the high S/N ratio and the high throughput. DUPONT AURELIE ET AL: "Nanoscale three-dimensional single particle tracking", NANOSCALE, vol. 3, no. 11, 1 January 2011 (2011-01-01), page 4532, discloses nanoscale single particle tracking for tracking a single biomolecule.

### SUMMARY OF THE INVENTION

According to a first aspect of the present invention, there is provided a measurement apparatus as specified in claims 1 to 13. According to a second aspect of the present invention, there is provided a sorting apparatus as specified in claims 14 and 15.

Further features of the present invention will become apparent from the following description of embodiments with reference to the attached drawings.

### BRIEF DESCRIPTION OF THE DRAWINGS

Fig. 1 is a diagram illustrating a sorting system.
Fig. 2 is a diagram illustrating details of a measurement unit.
Fig. 3 is a diagram illustrating a state where galvano scanners perform scanning with illumination light.
Fig. 4 is a diagram illustrating a state where a scanning area is scanned with illumination light.
Fig. 5 is a diagram illustrating a scanning locus drawn when a plurality of samples is measured in a scanning area.
Fig. 6 is a diagram illustrating a state where a sample arranged outside the scanning area is measured.

### DESCRIPTION OF THE EMBODIMENTS

An embodiment of the present invention will be described in detail with reference to the appended drawings.

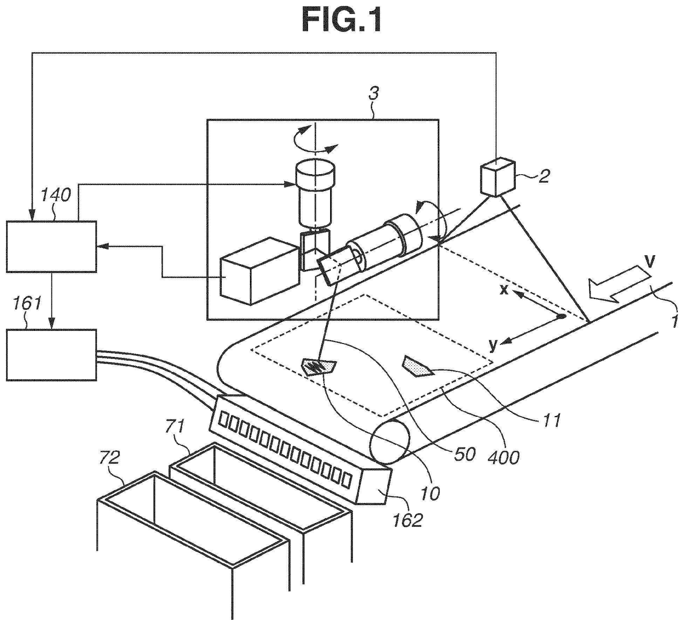

Fig. 1 is a diagram illustrating a configuration of a sorting system. The sorting system according to the present embodiment includes a conveyer-belt 1 (moving unit) for conveying a sample (object). The sorting system is a system which executes identification processing for identifying a type of a sample conveyed by the conveyer-belt 1 and sorts out a sample based on an identification result thereof.

Samples 10 and 11 are objects such as a piece of metal mainly containing metal, a piece of ceramic mainly containing metallic oxide crystals, a piece of glass mainly containing non-crystalline metallic oxides, and a piece of resin mainly containing resin. In many cases, the samples 10 and 11 are industrial waste such as discarded home electronics and waste automobiles, crushed to flat pieces having sizes of approximately 10 to 100 mm by crushing processing performed as the preprocessing of recycling processing.

In the present specification, "resin" generally refers to a polymer of organic materials including thermoplastic resin (plastic), thermosetting resin, rubber, elastomer, cellulose, and paper.

In addition, the samples 10 and 11 may contain various additive substances such as glass filler, fiber filler, a flame retardant, and a plasticizing agent.

The sorting system according to the present embodiment identifies not only a type of resin which constitutes a piece or resin, i.e., a type (material and color) of a piece of resin, but also presence or absence of the above-described additive substances and the type thereof.

In the sorting system according to the present embodiment, samples to be sorted are fed to the conveyer-belt 1 by a quantitative delivery device and a vibrating feeder (not illustrated) and conveyed on the conveyer-belt 1. A recognition unit (measuring unit) 2, a measurement unit 3, and a sorting unit 162 are arranged on the conveyer-belt 1 in that order from an upstream side of the moving direction of the conveyer-belt 1, and the sample 10 that has passed the lower side of the recognition unit 2 and the measurement unit 3 is dropped from the conveyer-belt 1 and passes across the front face of the sorting unit 162.

The recognition unit 2 executes image processing of a two-dimensional image including a sample captured by an area scan camera capable of collectively capturing a two-dimensional image or a line scan camera capable of reconstructing a two-dimensional image. For example, the recognition unit 2 calculates a coordinate p1(x1, y1) of a position where the sample 10 exists at a time t0 based on a result of the image processing and the information about a shape and a size of the sample 10.

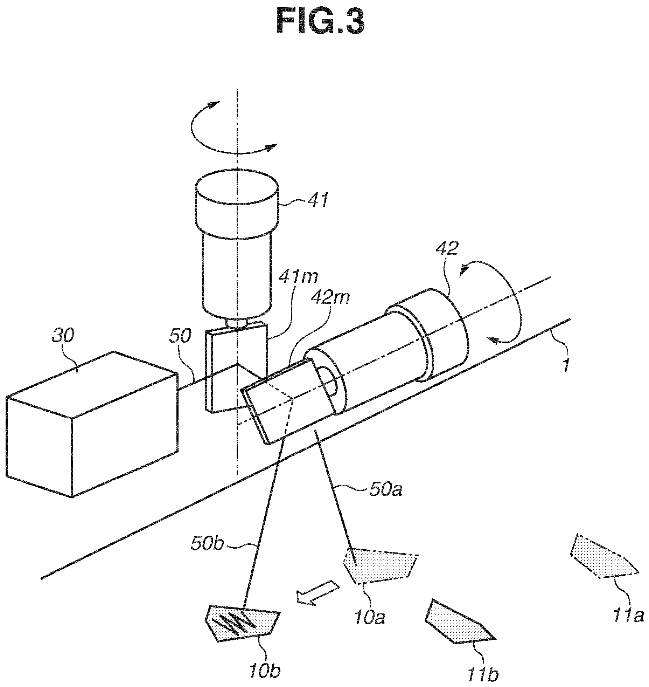

As illustrated in Fig. 3, the measurement unit 3 includes galvano scanners 41 and 42 (scanning unit) for performing scanning with illumination light 50, and an optical spectroscopic unit 30 for measuring Raman scattering light (reflection light) from a sample. The measurement unit 3 controls the scanning unit to incline the illumination light 50 at an angle as expressed by illumination light 50a or 50b by inclining a principal light ray of the illumination light 50, using the measurement unit 3 as a reference, and brings the illumination light (measurement point) into a movable state.

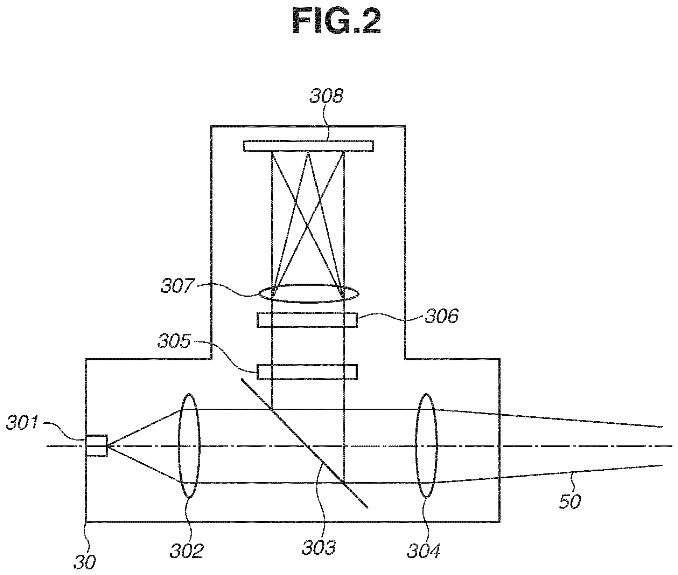

As illustrated in Fig. 2, the optical spectroscopic unit 30 includes a light source 301 and a lens (illumination unit) 302.

The optical spectroscopic unit 30 irradiates mirrors included in the galvano scanners 41 and 42 with laser light as the illumination light 50 from the light source 301 via lenses 302 and 304. A dichroic mirror 303 is arranged at a position between the lenses 302 and 304, and transmits light of a wavelength λ from the light source 301.

A processing unit 140 includes a control unit for controlling the galvano scanners 41 and 42. The processing unit 140 calculates a position coordinate p1(x1, y1 + V(t1 - t0)) of a moving sample at a time t1 from a speed V of the conveyer-belt 1 adjusted or measured previously and a time t0 at which the sample is recognized by the recognition unit 2. Then, at a time when the sample 10 reaches the scanning area 400 the galvano scanners 41 and 42 can scan with the illumination light 50, the processing unit 140 outputs control instructions to the galvano scanners 41 and 42 to control the galvano scanners 41 and 42 to illuminate the sample 10 with the illumination light 50. Then, the processing unit 140 controls the galvano scanners 41 and 42 to track the moving sample 10 with the illumination light 50. By using the two galvano scanners 41 and 42, scanning can be performed with illumination light 50 in the y axis direction, i.e., a moving direction of the conveyer-belt 1 (sample 10) and an x axis direction perpendicular to the y axis direction.

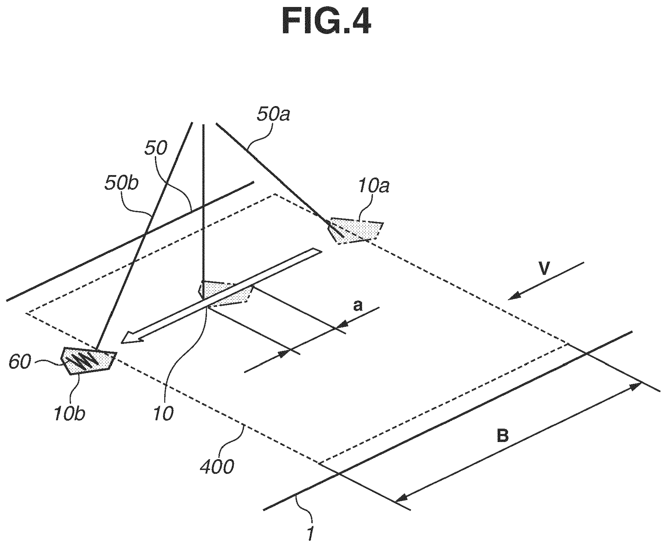

Specifically, as illustrated in Fig. 4, at a time point (position 10a) when the sample 10 reaches a boundary of the scanning area 400 on the upstream side in the moving direction of the conveyer-belt 1, the measurement unit 3 performs scanning with the illumination light 50 at a position of the illumination light 50a to start the measurement. The measurement unit 3 draws a locus 60 on the sample 10 to illuminate the sample 10 to perform the Raman measurement while the sample 10 moves at a speed V according to the movement of the conveyer-belt 1. Then, the measurement unit 3 completes the measurement when the sample 10 reaches a boundary (position 10b) of the scanning area 400 on the downstream side of the conveyer-belt 1. Herein, a locus from the illumination light 50a to the illumination light 50b expresses an average position of the illumination light 50 when scanning is performed.

A length of the scanning area 400 in a belt moving direction is expressed as "B". A position of the illumination light 50 changes in a direction the same as the belt moving direction, and the illumination light 50 illuminates the sample 10 for a time T (T = B/V) while tracking the sample 10 without staying at one point on a surface of the sample 10.

Therefore, energy of the illumination light 50 does not concentrate on one point of the sample 10, so that the measurement unit 3 can perform the Raman measurement without denaturalizing the resin. Further, because the conveyer-belt 1 can be operated at high speed, even if a huge amount of samples are fed to the conveyer-belt 1 for the sake of enhancing the throughput of identification, the samples can be arranged in a spatially separated state.

In the conventional method in which the illumination light 50 does not perform scanning, the sample 10 is irradiated for a time t (t = a/V) when a size of the sample 10 in the belt moving direction is "a".

The present embodiment will be described with respect to a case where a speed of the conveyer-belt 1 is 1 m/s, a size of the sample 10 assumed to be a minimum size in the recycling processing is 10 mm, and a scanning area B is set as 200 mm.

In the conventional method in which illumination light is fixed, the time t the sample can be illuminated with illumination light is 10 ms. On the other hand, in the method in which the measurement is executed while the sample is being tracked and scanned with the illumination light 50, measurement can be executed for approximately 200 ms. Therefore, with respect to black resin whose reflection light amount is small, a signal-noise ratio (S/N) of a faint Raman signal can be improved by extending the exposure time, or by averaging the data acquired by executing measurement for the exposure time of 10 ms twenty times.

On the other hand, in the present embodiment, while the sample 10 is being measured, the illumination light 50 draws a zigzag locus, such as a locus 60, without staying at one place. Therefore, energy of the illumination light 50 is widely dispersed in the area of the sample 10, so that the resin is not denaturalized while measurement is being executed.

As described above, the illumination light 50 from the optical spectroscopic unit 30 is reflected on the mirror portions 41m and 42m of the galvano scanners 41 and 42 and irradiate the sample 10.

After the illumination light 50 is reflected on the sample 10, Raman scattering light is reflected isotropically, reflected on the mirror portions 41m and 42m of the galvano scanners 41 and 42 again, and returns to the optical spectroscopic unit 30. The Raman scattering light is Raman-shifted depending on the substances of the material of the sample 10. Therefore, the Raman scattering light is subsequently reflected on the dichroic mirror 303, and only the components thereof which have been Raman-shifted are transmitted through a bandpass filter 305 serving as an excitation light cut filter and dispersed by a spectroscopic element 306 (spectroscopic unit). The light flux dispersed by the spectroscopic element 306, diffracted by a diffraction angle different for each wavelength, is condensed again through a lens 307, and Raman-shifted light of each wavelength is received by a light receiving element arranged on the sensor array 308. A signal output from the light receiving element is transmitted to the processing unit 140 as a characteristic signal depending on the substance of the sample. Then, for example, the processing unit 140 compares a waveform thereof with a unique waveform of a known material previously acquired by measuring a sample of the known material to specify the material, and identifies a type of the sample.

For example, a signal (measurement result) from the sensor array 308 is transmitted to the processing unit 140 at 300 fps. The measurement is started at a time point when the sample 10 reaches the position 10a, i.e., a boundary of the scanning area 400 on the upstream side in the belt moving direction. Then, the processing unit 140 extracts a signal corresponding to that measurement from a group of signals transmitted from the sensor array 308 at 300 fps and specifies a material. For example, a corresponding signal may be determined and extracted based on a time the operation instruction is issued.

In a case where the processing unit 140 identifies the type of the sample 10 and determines that the sample 10 is a sorting target material, an opening signal is transmitted to the sorting unit 162 via the sorting controller 161 at a time t2 when the sample 10 passes the sorting unit 162. Then, air is spouted from a portion of the sorting unit 162 at a position corresponding to a coordinate x1 of the sample 10 measured by the recognition unit 2, so that the sample 10 is blown off by the air and collected to a collection box 72. In a case where the sample 10 is not a sorting target, the sample 10 is thrown out and dropped from the conveyer-belt 1 at a speed V and collected to a collection box 71.

After measurement of the sample 10 is completed, the measurement unit 3 executes measurement of the sample 11 and subsequent samples (not illustrated) which are continuously fed and conveyed thereto. The measurement unit 3 turns the galvano scanners 41 and 42 to change the inclination angle of the illumination light 50 to move the illumination light 50 to a starting point for executing measurement of the sample 11. Because the galvano scanners 41 and 42 can rapidly perform scanning with the illumination light 50, a moving time until measuring the next resin can be shortened. Therefore, the galvano scanners 41 and 42 can continuously measure the samples conveyed one after another. As a result, the number of samples measured by the Raman measurement with long measurement time and high S/N ratio is increased, so that resin can be sorted with high throughput.

In the present embodiment, the recognition unit 2 acquires a two-dimensional image captured by an area sensor or a line sensor, and calculates a coordinate of a sample through the image processing. However, the configuration is not limited thereto. For example, an object may be recognized based on the height information acquired by a light-sectioning sensor. Further, in the present embodiment, a zigzag locus has been described as a locus which the illumination light 50 draws on resin without stopping at one point thereof. However, it is obvious that the same effect can be acquired as long as the illumination light 50 does not stop at one point on resin. For example, the illumination light 50 may continuously make a circular or helical motion, or may draw a square-shape locus.

Further, according to the configuration described in the present embodiment, the recognition unit 2 acquires a two-dimensional image which includes a sample. This two-dimensional image includes brightness of the sample, i.e., information about diffuse reflectivity. According to the study conducted by those skilled in the art, it was found that light intensity of the Raman scattering correlates with diffuse reflectivity, and that intensity of the Raman scattering light coming from a white sample having high brightness is much higher than the Raman scattering light coming from a black sample having low brightness. In other words, a measurement time of the white sample necessary to acquire the same S/N ratio can be shorter than that of the black sample.

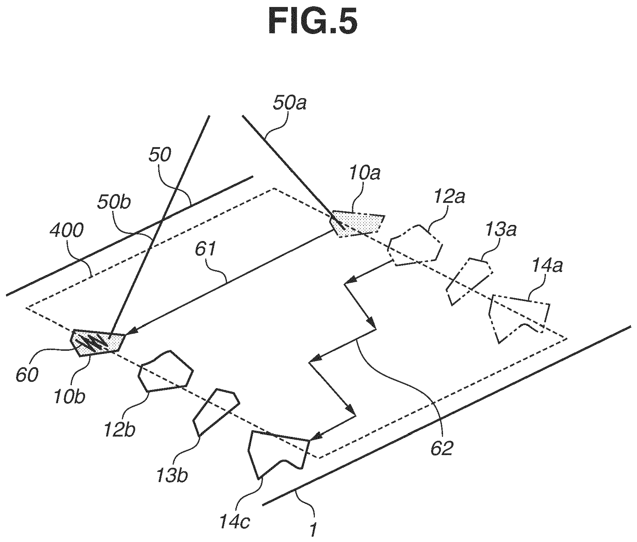

In view of the above-described fact, in order to efficiently execute identification of the sample for the sake of improving the throughput, it is effective to make the measurement time of the white resin be shorter than the measurement time of the black resin. In this case, as illustrated in Fig. 5, with respect to the sample 10 which is black resin, the galvano scanners 41 and 42 perform scanning with the illumination light 50 under the scanning condition that measurement is executed along a locus 61 which is drawn across the entire scanning area 400 from the position 10a to the position 10b.

On the other hand, with respect to the white resin, for example, scanning is performed with illumination light 50 under the scanning condition that measurement is executed in a measurement time one-third the measurement time of the black resin. For example, scanning is performed with the illumination light 50 along a step-like locus 62. In this way, with respect to samples 12, 13, 14 having high brightness, conveyed side by side from a position 12a to a position 12b, a position 13a to a position 13b, and a position 14a to a position 14b, the measurement unit 3 can measure all of these three samples 12, 13, and 14. In this case, the loci 61 and 62 illustrate the average positions of the illumination light 50 when scanning is performed, and it is needless to say that the illumination light 50 simultaneously draws the locus 60 on the resin while the average positions thereof are being moved on the respective loci 61 and 62. As described above, the processing unit 140 includes a control unit which controls the scanning unit to change the scanning condition of illumination light 50 based on the information about the object.

In addition, samples conveyed thereto are different in size and color. As described above, the higher the brightness of the sample is, the higher the Raman scattering intensity becomes, and the lower the brightness of the sample is, the lower the Raman scattering intensity becomes. Therefore, the higher the brightness is, the higher the identification reliability of the specified resin type, and the lower the brightness is, the lower the identification reliability of the specified resin type. It is obvious that a utility value of a desired type of collected resin is increased when its purity is high. Further, it is preferable that resin having higher brightness be collected preferentially because the identification reliability is improved, and purity of a desired type of collected resin is increased as well.

On the other hand, the samples existing in the scanning area 400 are actually conveyed by the conveyer-belt 1 in a random arrangement. Therefore, there is a possibility that the samples exist in high distribution density. In such a case, there is not enough time to sequentially scan and measure the entire group of resin in high distribution density with the illumination light 50. In this case, the processing unit 140 may instruct the galvano scanners 41 and 42 to skip the measurement of some samples. In other words, in a case where a plurality of objects exists in an area scannable with the illumination light 50, the processing unit 140 determines a measurement target object based on the information about the object.

At this time, the samples having high brightness are measured preferentially based on the information about brightness of respective samples captured in the image acquired by the recognition unit 2. In this way, it is possible to improve the purity of collected samples. Further, it is also effective to increase the amount of collected resin in the end by preferentially measuring the samples having large size, based on the information about a shape of the sample acquired by the recognition unit 2.

According to the configuration described in the present embodiment, the processing unit 140 sets the previously adjusted or measured speed V as a speed of the conveyer-belt 1 and issues an instruction for causing the measurement unit 3 to track the sample with the illumination light 50 and an instruction for causing the sorting unit 162 to perform the sorting operation based on time. However, for example, measuring devices such as a laser Doppler velocimeter and an image correlation displacement meter, which directly measure a speed of the conveyer-belt 1, may be arranged in the peripheries of the recognition unit 2 and the measurement unit 3, so that the processing unit 140 can receive the measurement values relating to the belt moving amount from these measuring devices. With this configuration, the processing unit 140 can issue the operation instructions to the galvano scanners 41 and 42 based on the belt moving amount measured thereby. It is effective to perform the tracking operation with the illumination light 50 more precisely and to perform sorting operation by the sorting unit 162 more precisely to increase the collection amount. In this case, as a method for extracting a signal generated at a time when a sample is illuminated, from a group of signals transmitted from the sensor array 308, the processing unit 140 can determine the signal by using a measurement value relating to the belt moving amount acquired when each signal is generated. In this way, the processing unit 140 can precisely extract a signal generated when the illumination light 50 exists on the resin.

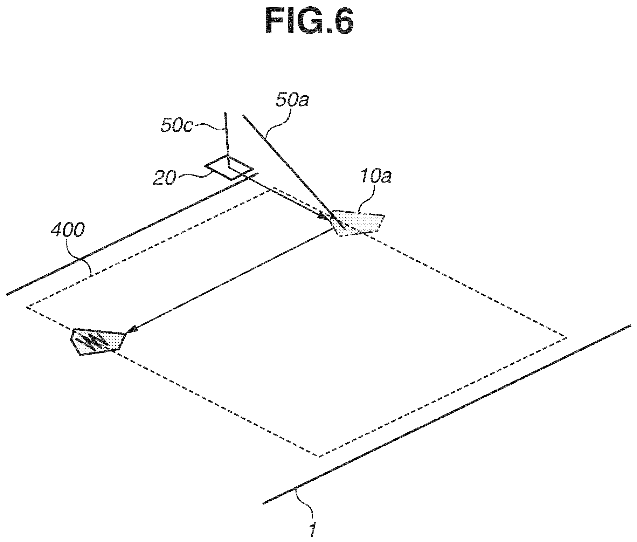

With respect to extraction of the signal, it is also effective to measure a sample which exhibits a known specific characteristic (feature) not assumed in the normal measurement in advance, prior to the specified time or immediately before the illumination light 50 moves to the measurement starting point. For example, a sample having high reflectivity, whose measurement value is saturated, may be measured previously. For example, as illustrated in Fig. 6, a fluorescent sample 20 having high fluorescence, whose measurement value is saturated at the sensor array 308, is arranged at a position outside the scanning area 400 and where the measurement unit 3 can perform measurement. Then, prior to the specified time or immediately before the sample 10 that has reached the position 10a on the border of the scanning area 400 on the upstream side in the belt moving direction is scanned with the illumination light 50, the measurement unit 3 illuminates the fluorescent sample 20 by changing the angle of the illumination light 50 to an angle expressed by an illumination light 50c and measures the fluorescent sample 20. In this way, in the subsequent stage, the processing unit 140 can easily extract the measurement data. As the data to be used for determining a material of the sample, the processing unit 140 may extract, from the data continuously transferred from the sensor array 308, data transferred immediately after data indicating the element having a maximum signal value or data transferred after the specified number of pieces of data, and executes the processing.

The sample which indicates the characteristic data is not limited to the above, and any sample which exhibits a known specific characteristic, not assumed in the normal measurement, can be used. For example, the sample may be a light trap having an extremely small amount of reflection light, or resin alloy which simultaneously exhibits characteristics of a plurality of types of resin.

As described above, based on the information about the resin recognized by the recognition unit 2, the galvano scanners 41 and 42 described as an example of the scanning unit makes the average positions follow the belt moving direction to illuminate the sample with the illumination light 50 by changing the principal light ray angle of the illumination light 50. Further, while the measurement is being executed, even if the belt speed is high, the measurement unit 3 tracks the sample to execute point-measurement through the Raman spectroscopy by performing scanning with the illumination light 50 without making the illumination light 50 stay at the same position on the resin. Therefore, the measurement unit 3 can identify the resin through the Raman spectroscopy while achieving both of the high S/N and the high throughput.

According to the present embodiment, it is possible to provide an identification apparatus capable of achieving both of the high S/N ratio and the high throughput.

While the present invention has been described with reference to embodiments, it is to be understood that the invention is not limited to the disclosed embodiments but is defined by the scope of the following claims.

## Claims

1. A measurement apparatus (3), comprising:
scanning means (41, 42) configured to move illumination light to illuminate an object;
control means (140) configured to control the scanning means (41, 42); and
an optical spectroscopic unit (30) configured to measure reflection light from the illuminated object,
wherein the optical spectroscopic unit (30) includes spectroscopic means (306) configured to disperse scattering light from the object illuminated by the illumination light,
**characterized in that**:
the control means (140) is configured to control the scanning means (41, 42) so that the illumination light from the scanning means to illuminate the object tracks the moving object by changing a position of the illumination light in a moving direction of the object and a direction perpendicular to the moving direction.

2. The measurement apparatus according to claim 1, wherein, the control means (140) is configured to control the scanning means (41, 42) to prevent the illumination light from staying at one point on a surface of the object while the moving object is being measured.

3. The measurement apparatus according to claim 1, wherein the control means (140) is configured to control the scanning means (41, 42) to perform scanning with the illumination light in the direction perpendicular to the moving direction while performing scanning with the illumination light in the moving direction of the object.

4. The measurement apparatus according to claim 1, wherein the control means (140) is configured to control the scanning means (41, 42) to change how scanning of the illumination light is performed based on information about the object.

5. The measurement apparatus according to claim 1, wherein, in a case where a plurality of objects exists in an area scannable with the illumination light, the control means (140) is configured to determine a measurement target object based on information about the object.

6. The measurement apparatus according to claim 5, wherein the information about the object includes brightness or size of the object.

7. The measurement apparatus according to claim 1, wherein the measurement apparatus is configured to identify a type of the object by using data previously obtained by measuring samples by the optical spectroscopic unit (30).

8. The measurement apparatus according to claim 1, further comprising measurement means for measuring a position or a speed of the moving object,
wherein the control means controls the scanning means based on the position or the speed of the object measured by the measurement means while the object is being illuminated by illumination light.

9. The measurement apparatus according to claim 1, wherein the measurement apparatus is configured to identify a type of resin as the object.

10. The measurement apparatus according to claim 1, wherein the scanning means (41, 42) includes a galvano scanner.

11. The measurement apparatus according to claim 1, wherein the control means is configured to control the scanning means (41, 42) based on a position of the object to make the scanning means track the moving object with the illumination light and change a position of the illumination light on a surface of the object.

12. The measurement apparatus according to claim 1, further comprising recognition means for recognizing a position of an object,
wherein, based on a position of the object obtained from the recognition means, the control means makes the scanning means perform scanning with the illumination light.

13. The measurement apparatus according to claim 1, wherein the control means controls the scanning means so that the illumination light from the scanning means to illuminate the object tracks the moving object, and a position of the illumination light illuminated to the object to move in two directions perpendicular to each other.

14. The measurement apparatus according to claim 13, wherein the scanning means (41, 42) includes a plurality of galvano scanners configured to move the position of the illumination light in two directions perpendicular to each other.

15. A sorting apparatus for sorting an object, comprising:
a measurement apparatus,
wherein the measurement apparatus (3), comprises:
scanning means (41, 42) configured to move illumination light to illuminate an object;
control means (140) configured to control the scanning means (41, 42); and
an optical spectroscopic unit (30) configured to measure reflection light from the illuminated object,
wherein the control means (140) is configured to control the scanning means so that the illumination light from the scanning means to illuminate the object tracks the moving object by changing a position of the illumination light in a moving direction of the object and a direction perpendicular to the moving direction,
**characterized in that**:
the sorting apparatus further comprises sorting means configured to sort the object based on a measurement result obtained by the measurement apparatus.

16. The sorting apparatus according to claim 15, further comprising moving means for moving the object.

## Patentansprüche

1. Messvorrichtung (3), umfassend:
eine Abtasteinrichtung (41, 42), die konfiguriert ist, Beleuchtungslicht zu bewegen, um ein Objekt zu beleuchten;
eine Steuereinrichtung (140), die konfiguriert ist, die Abtasteinrichtung (41, 42) zu steuern; und
eine Optische-Spektroskopie-Einheit (30), die konfiguriert ist, Reflexionslicht von dem beleuchteten Objekt zu messen,
wobei die Optische-Spektroskopie-Einheit (30) eine Spektroskopieeinrichtung (306) enthält, die konfiguriert ist, Streulicht von dem durch das Beleuchtungslicht beleuchteten Objekt spektral zu zerlegen,
**dadurch gekennzeichnet, dass**:
die Steuereinrichtung (140) konfiguriert ist, die Abtasteinrichtung (41, 42) so zu steuern, dass das Beleuchtungslicht von der Abtasteinrichtung zum Beleuchten des Objekts dem sich bewegenden Objekt folgt, indem eine Position des Beleuchtungslichts in einer Bewegungsrichtung des Objekts und einer Richtung senkrecht zur Bewegungsrichtung geändert wird.

2. Messvorrichtung nach Anspruch 1, wobei die Steuereinrichtung (140) konfiguriert ist, die Abtasteinrichtung (41, 42) zu steuern, um zu verhindern, dass das Beleuchtungslicht an einem Punkt auf einer Oberfläche des Objekts bleibt, während das sich bewegende Objekt gemessen wird.

3. Messvorrichtung nach Anspruch 1, wobei die Steuereinrichtung (140) konfiguriert ist, die Abtasteinrichtung (41, 42) zu steuern, um ein Abtasten mit dem Beleuchtungslicht in der Richtung senkrecht zur Bewegungsrichtung durchzuführen, während ein Abtasten mit dem Beleuchtungslicht in der Bewegungsrichtung des Objekts durchgeführt wird.

4. Messvorrichtung nach Anspruch 1, wobei die Steuereinrichtung (140) konfiguriert ist, die Abtasteinrichtung (41, 42) zu steuern, um basierend auf Informationen über das Objekt zu ändern, wie das Abtasten des Beleuchtungslichts durchgeführt wird.

5. Messvorrichtung nach Anspruch 1, wobei in einem Fall, in dem mehrere Objekte in einem mit dem Beleuchtungslicht abtastbaren Bereich vorhanden sind, die Steuereinrichtung (140) konfiguriert ist, ein Messzielobjekt basierend auf Informationen über das Objekt zu bestimmen.

6. Messvorrichtung nach Anspruch 5, wobei die Informationen über das Objekt die Helligkeit oder Größe des Objekts enthalten.

7. Messvorrichtung nach Anspruch 1, wobei die Messvorrichtung konfiguriert ist, einen Typ des Objekts unter Verwendung von Daten zu identifizieren, die zuvor durch Messen von Proben durch die Optische-Spektroskopie-Einheit (30) erhalten wurden.

8. Messvorrichtung nach Anspruch 1, ferner umfassend eine Messeinrichtung zum Messen einer Position oder einer Geschwindigkeit des sich bewegenden Objekts,
wobei die Steuereinrichtung die Abtasteinrichtung basierend auf der Position oder der Geschwindigkeit des Objekts steuert, die durch die Messeinrichtung gemessen wird, während das Objekt durch Beleuchtungslicht beleuchtet wird.

9. Messvorrichtung nach Anspruch 1, wobei die Messvorrichtung konfiguriert ist, einen Typ von Harz als das Objekt zu identifizieren.

10. Messvorrichtung nach Anspruch 1, wobei die Abtasteinrichtung (41, 42) einen Galvanoscanner enthält.

11. Messvorrichtung nach Anspruch 1, wobei die Steuereinrichtung konfiguriert ist, die Abtasteinrichtung (41, 42) basierend auf einer Position des Objekts zu steuern, um zu bewirken, dass die Abtasteinrichtung dem sich bewegenden Objekt mit dem Beleuchtungslicht folgt, und eine Position des Beleuchtungslichts auf einer Oberfläche des Objekts zu ändern.

12. Messvorrichtung nach Anspruch 1, ferner umfassend eine Erkennungseinrichtung zum Erkennen einer Position eines Objekts,
wobei die Steuereinrichtung basierend auf einer Position des Objekts, die von der Erkennungseinrichtung erhalten wird, bewirkt, dass die Abtasteinrichtung ein Abtasten mit dem Beleuchtungslicht durchführt.

13. Messvorrichtung nach Anspruch 1, wobei die Steuereinrichtung die Abtasteinrichtung so steuert, dass das Beleuchtungslicht von der Abtasteinrichtung zum Beleuchten des Objekts dem sich bewegenden Objekt folgt, und eine Position des Beleuchtungslichts, das das Objekt beleuchtet, sich in zwei Richtungen senkrecht zueinander bewegen.

14. Messvorrichtung nach Anspruch 13, wobei die Abtasteinrichtung (41, 42) mehrere Galvanoscanner enthält, die konfiguriert sind, die Position des Beleuchtungslichts in zwei Richtungen senkrecht zueinander zu bewegen.

15. Sortiervorrichtung zum Sortieren eines Objekts, umfassend:
eine Messvorrichtung,
wobei die Messvorrichtung (3) umfasst:
eine Abtasteinrichtung (41, 42), die konfiguriert ist, Beleuchtungslicht zu bewegen, um ein Objekt zu beleuchten;
eine Steuereinrichtung (140), die konfiguriert ist, die Abtasteinrichtung (41, 42) zu steuern; und
eine Optische-Spektroskopie-Einheit (30), die konfiguriert ist, Reflexionslicht von dem beleuchteten Objekt zu messen,
wobei die Steuereinrichtung (140) konfiguriert ist, die Abtasteinrichtung so zu steuern, dass das Beleuchtungslicht von der Abtasteinrichtung zum Beleuchten des Objekts dem sich bewegenden Objekt folgt, indem eine Position des Beleuchtungslichts in einer Bewegungsrichtung des Objekts und einer Richtung senkrecht zur Bewegungsrichtung geändert wird,
**dadurch gekennzeichnet, dass**:
die Sortiervorrichtung ferner eine Sortiereinrichtung umfasst, die konfiguriert ist, das Objekt basierend auf einem von der Messvorrichtung erhaltenen Messergebnis zu sortieren.

16. Sortiervorrichtung nach Anspruch 15, ferner umfassend eine Bewegungseinrichtung zum Bewegen des Objekts.

## Revendications

1. Appareil de mesure (3), comprenant :
des moyens de balayage (41, 42) configurés pour déplacer une lumière d'éclairage afin d'éclairer un objet ;
des moyens de commande (140) configurés pour commander les moyens de balayage (41, 42) ; et
une unité spectroscopique optique (30) configurée pour mesurer une lumière réfléchie par l'objet éclairé,
dans lequel l'unité spectroscopique optique (30) inclut des moyens spectroscopiques (306) configurés pour disperser une lumière diffusée provenant de l'objet éclairé par la lumière d'éclairage,
**caractérisé en ce que** :
les moyens de commande (140) sont configurés pour commander les moyens de balayage (41, 42) de sorte que la lumière d'éclairage provenant des moyens de balayage pour éclairer l'objet suive l'objet en déplacement en modifiant une position de la lumière d'éclairage dans une direction de déplacement de l'objet et dans une direction perpendiculaire à la direction de déplacement.

2. Appareil de mesure selon la revendication 1, dans lequel les moyens de commande (140) sont configurés pour commander les moyens de balayage (41, 42) afin d'empêcher la lumière d'éclairage de rester à un point sur une surface de l'objet pendant que l'objet en déplacement est mesuré.

3. Appareil de mesure selon la revendication 1, dans lequel les moyens de commande (140) sont configurés pour commander les moyens de balayage (41, 42) afin de réaliser un balayage avec la lumière d'éclairage dans la direction perpendiculaire à la direction de déplacement tout en réalisant un balayage avec la lumière d'éclairage dans la direction de déplacement de l'objet.

4. Appareil de mesure selon la revendication 1, dans lequel les moyens de commande (140) sont configurés pour commander les moyens de balayage (41, 42) afin de modifier la manière dont le balayage de la lumière d'éclairage est réalisé sur la base d'informations concernant l'objet.

5. Appareil de mesure selon la revendication 1, dans lequel, dans le cas où il existe une pluralité d'objets dans une zone pouvant être balayée par la lumière d'éclairage, les moyens de commande (140) sont configurés pour déterminer un objet cible de mesure sur la base d'informations concernant l'objet.

6. Appareil de mesure selon la revendication 5, dans lequel les informations concernant l'objet incluent une luminosité ou une taille de l'objet.

7. Appareil de mesure selon la revendication 1, dans lequel l'appareil de mesure est configuré pour identifier un type de l'objet à l'aide de données préalablement obtenues en mesurant des échantillons par l'unité spectroscopique optique (30).

8. Appareil de mesure selon la revendication 1, comprenant en outre des moyens de mesure pour mesurer une position ou une vitesse de l'objet en déplacement,
dans lequel les moyens de commande commandent les moyens de balayage sur la base de la position ou de la vitesse de l'objet mesurée par les moyens de mesure pendant que l'objet est éclairé par une lumière d'éclairage.

9. Appareil de mesure selon la revendication 1, dans lequel l'appareil de mesure est configuré pour identifier un type de résine en tant qu'objet.

10. Appareil de mesure selon la revendication 1, dans lequel les moyens de balayage (41, 42) comprennent un scanner galvanométrique.

11. Appareil de mesure selon la revendication 1, dans lequel les moyens de commande sont configurés pour commander les moyens de balayage (41, 42) sur la base de la position de l'objet afin d'amener les moyens de balayage à suivre l'objet en déplacement avec la lumière d'éclairage et de modifier une position de la lumière d'éclairage sur une surface de l'objet.

12. Appareil de mesure selon la revendication 1, comprenant en outre des moyens de reconnaissance pour reconnaître une position d'un objet,
dans lequel, sur la base d'une position de l'objet obtenue à partir des moyens de reconnaissance, les moyens de commande amènent les moyens de balayage à réaliser un balayage avec la lumière d'éclairage.

13. Appareil de mesure selon la revendication 1, dans lequel les moyens de commande commandent les moyens de balayage de sorte que la lumière d'éclairage émise par les moyens de balayage pour éclairer l'objet suive l'objet en déplacement, et une position de la lumière d'éclairage projetée sur l'objet se déplace dans deux directions perpendiculaires l'une à l'autre.

14. Appareil de mesure selon la revendication 13, dans lequel les moyens de balayage (41, 42) incluent une pluralité de scanners galvanométriques configurés pour déplacer la position de la lumière d'éclairage dans deux directions perpendiculaires l'une à l'autre.

15. Appareil de tri pour trier un objet, comprenant :
un appareil de mesure,
dans lequel l'appareil de mesure (3) comprend :
des moyens de balayage (41, 42) configurés pour déplacer une lumière d'éclairage afin d'éclairer un objet ;
des moyens de commande (140) configurés pour commander les moyens de balayage (41, 42) ; et
une unité spectroscopique optique (30) configurée pour mesurer une lumière réfléchie par l'objet éclairé,
dans lequel les moyens de commande (140) sont configurés pour commander les moyens de balayage de sorte que la lumière d'éclairage émise par les moyens de balayage pour éclairer l'objet suive l'objet en déplacement en modifiant une position de la lumière d'éclairage dans une direction de déplacement de l'objet et dans une direction perpendiculaire à la direction de déplacement,
**caractérisé en ce que** :
l'appareil de tri comprend en outre des moyens de tri configurés pour trier l'objet sur la base d'un résultat de mesure obtenu par l'appareil de mesure.

16. Appareil de tri selon la revendication 15, comprenant en outre des moyens de déplacement pour déplacer l'objet.
